# EUROPEAN PATENT SPECIFICATION

(11) **EP 0 608 952 B1**
(45) Date of publication and mention of the grant of the patent: **07.10.1998**
(21) Application number: 94200175.1
(22) Date of filing: 26.01.1994
(51) Int. Cl.: C07C 39/23, C07C 39/17, C07D 303/24, C08G 59/32, C08G 61/02

(54) **Phenolic compounds and glycidyl ethers thereof**
Phenolische Verbindungen sowie ihre Glycidylether
Composés phénoliques ainsi que leurs éthers glycidyliques

(30) Priority: 28.01.1993 US 10128; 05.02.1993 US 13837
(43) Date of publication of application: 03.08.1994
(73) Proprietor: SHELL INTERNATIONALE RESEARCH MAATSCHAPPIJ B.V., 2596 HR Den Haag (NL)
(72) Inventor: Wang, Pen Chung, Houston, Texas 77079 (US); Kelsey, Donald Ross, Fulshear, Texas 77441 (US); Corley, Larry Steven, Houston, Texas 77083 (US)

(56) References cited:
- DE-A- 3 533 488
- DE-A- 4 210 129
- GB-A- 2 249 095
- US-A- 3 536 734
- US-A- 5 095 082

## Description

This invention relates to novel phenolic compounds, per se suitable for use as curing agents for epoxy resins, and to glycidyl ether derivatives thereof.

Polyhydric phenol compounds as well as the corresponding glycidyl ether derivatives are known. For instance, in DE 4,210,129 polyhydric phenol compounds are described which are prepared by the reaction of a phenol compound and a tetrahydroindene compound.

In US 3,536,734, DE 3,533,488 and GB 2,249,095 polyhydric phenol compounds are described derived from phenol compounds and dicyclopentadiene. The polyhydric compounds described in the above-mentioned documents can be used as curing agents in epoxy resin composition or can be used as starting compounds for glycidyl ether compounds by reaction with epichlorohydrin in the presence of a hydroxide.

It is known that epoxy resin compositions have wide structural-, coating- and electronic applications. For high-performance electronic applications, such as circuit boards for high-speed computers, epoxy resin compositions, having increasingly low melt viscosity and low water absorbance in the cured state, are required (for ease and speed of processing during the prepregging stage of electrical lamination preparation).

Therefor in epoxy resin-based electrical lamination formulations, it is desirable for both the epoxy resin and the curing agent to have a low melt viscosity.

Thus the problem underlying the present invention can be seen in providing novel phenolic and epoxy resin compounds having a low melt viscosity.

The present invention relates to a phenolic compound of the formula I 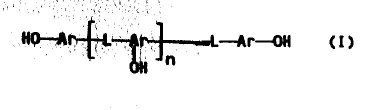 in which Ar is a C₆₋₂₀ aromatic group, L is a divalent cyclohexanenorbornane moiety, and n is a number of from 0 to 10.

Preferred embodiments of said phenolic compounds of formula I are those wherein Ar is phenyl and L is selected from at least one of the groups represented by the following structural formula:

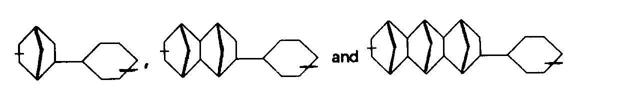

Another aspect of the present invention is formed by a composition comprising an epoxy resin and the hereinbefore specified phenolic compounds.

Another aspect of the present invention is formed by a process for preparing a phenolic compound as specified hereinbefore by contacting in a reaction mixture at least one cyclohexenenorbornene compound with a molar excess, with respect to the cyclohexenenorbornene, of at least one phenolic compound in the presence of a Lewis acid addition catalyst at a temperature within the range of 70 to 200 °C.

According to a preferred embodiment of said process, 5-(3-cyclohexen-t-yl) bicylo[2.2.1.]hept-2-ene is used as cyclohexenenorbornene compound.

The invention also relates to an epoxy resin of the formula II 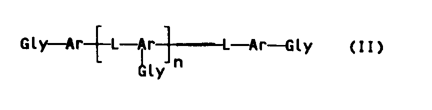 in which Gly is a glycidyl ether group, Ar is a C₆₋₂₀ aromatic moiety, L is a divalent cyclohexanenorbornane linking moiety, and n is a number within the range of 0 to 10.

Preferred embodiments of these of these epoxy resins are those, wherein Ar is phenyl and wherein l is selected from at least one of the groups represented by the following structural formula

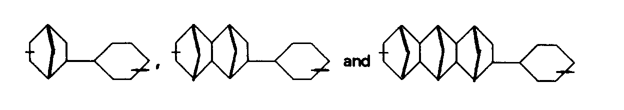

Another aspect of the invention is formed by an epoxy resin composition, comprising an hereinbefore specified epoxy resin and an effective amount of a curing agent for the epoxy resin.

According to a preferred embodiment of said composition the curing agent is diaminodiphenyl sulfone or methylenediamine.

The invention is also relating a cured product obtainable by subjecting an epoxy resin composition comprising the specific epoxy resins and a curing agent to a temperature of at least 150 °C for at least 0.25 hour and to articles comprising said cured product.

The polyphenols according to the present invention can be prepared by the addition reaction of a phenol with a cyclohexenenorbornene compound such as 5-(3-cyclohexen-1-yl)bicyclo[2.2.1]hept-2-ene. Suitable phenols include mono- and polynuclear phenols having at least one unsubstituted position ortho- or para- to a phenolic hydroxyl group, such as phenol, cresol, 3,4- and 3,5-dimethylphenol, resorcinol, biphenol, 1-naphthol and bisphenol A or F. Phenol is preferred. Suitable cyclohexenenorbornene compounds include 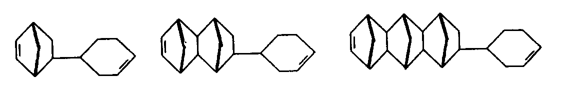 referred to herein as "monoadduct," "diadduct" and "triadduct," respectively, and isomers thereof.

The cyclohexenenorbornene itself is an adduct of 4-vinylcyclohexene and cyclopentadiene which can be prepared by contacting 4-vinylcyclohexene and dicyclopentadiene, preferably in the presence of a polymerization inhibitor such as t-butyl catechol, at a temperature of at least 180 °C, preferably of from 220 to 260 °C, for a time of from 2 to 8 hours. Under these conditions, the dicyclopentadiene is cracked to cyclopentadiene, and the vinylcyclohexene and cyclopentadiene undergo an addition reaction to produce a mixture of mono-, di- and poly-adducts along with cyclopentadiene oligomers (e.g., trimer, tetramer, pentamer, etc.). The reaction product mixture containing predominantly 5-(3-cyclohexen-1-yl)-2-norbornene (monoadduct) is allowed to cool to 50-70 °C and is stirred under reduced pressure to strip off unreacted vinylcyclohexene. The reaction product is then purified by fractional vacuum distillation to remove by-products including, optionally, di- and poly-adducts and cyclopentadiene oligomers, and the purified product is passed through an adsorent bed for removal of t-butyl catechol.

The reaction between the phenol and the cyclohexenenorbornene and optionally the cyclic diene is generally carried out by contacting, under addition reaction conditions, the cyclohexenenorbornene and optionally the cyclic diene, with a molar excess, preferably from 10 to 30 moles, of the selected phenol per mole of the cyclohexenenorbornene plus diene. The above reaction is most efficiently carried out in the presence of a Lewis acid addition catalyst such as BF₃, coordination complexes thereof such as boron trifluoride etherate, AlCl₃, FeCl₃, SnCl₄, ZnCl₂, silica and silica-alumina complexes and at an elevated temperature of from 70 to 200 °C, preferably of from 100 to 180 °C. The reaction is continued until the desired degree of reaction has been completed, usually for a period of 30 minutes to 10 hours. Normally a period of from 1 hour to 3 hours will be sufficient.

Examples 1, 2, and 3 further illustrate the preparation of polyphenols.

The phenolic compound can be combined with an epoxy resin by, for example, melt-blending, preferably in the presence of a curing catalyst such as an imidazole. Subsequent cure of the epoxy resin is effected by heating the epoxy/phenol mixture at a temperature higher than 150 °C, preferably of from 200 to 300 °C, for at least 0.25 hour. Cure of epoxy resins with phenols according to the present invention is illustrated in Examples 4, 5 and 6 herein.

As indicated above the polyphenols of the present invention are particularly suitable as curing agents for epoxy resins. Such epoxy resin compositions are useful in moulding powder-, coating- and electrical encapsulation and laminating applications.

In addition the polyphenols of the present invention may also be used as stabilizing additives for thermoplastics and as precursors for thermosettable resins.

In particular they were found to be suitable as precursors for epoxy resins compounds.

Accordingly the present invention relates to epoxy resin compounds of the formula II 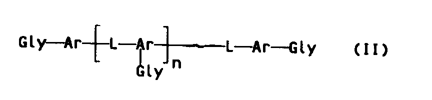 wherein L, m and n are as defined above and Gly is a glycidyl ether group.

The epoxy resin compounds according to the present invention can be prepared by reacting the precursor polyphenols of the formula I as described above with an epihalohydrin such as epichlorohydrin in the presence of a catalyst such as a quaternary ammonium salt or phosphonium halide, followed by dehydrochlorination under reduced pressure in the presence of aqueous caustic. The reaction can be carried out at a temperature within the range of about 40 to about 120 °C, preferably about 80 to about 110 °C.

Glycidation of the polyphenols according to the present invention to prepare the epoxy resin compounds is described in Example 7 herein.

The epoxy resin compounds of the formula II can be combined with a curing agent at a temperature of from 150 to about 250 °C for a period of time which can vary widely depending on the cure schedule and thickness of the part, which is generally greater than 0.25 hour. Suitable curing agents include amines such as diaminodiphenyl sulfone and methylene dianiline, and phenols such as phenolic novolacs and the precursor phenols. Optimum properties of the cured resin can be achieved by a staged heating process employing higher temperature in each stage. The epoxy resins can be co-cured with other thermosettable resins such as for example bismaleimides and cyanate esters.

Since the epoxy resin compounds of the formula II have in particular low melt viscosity and low water absorption in the cured state they are particularly suitable for use in electrical laminates, structural composites and moulding compounds.

### Example 1

### Preparation of Polyphenol Based on 5-(3-cyclohexen-1-yl)bicyclo[2.2.1]hept-2-ene

To a reactor equipped with a stirrer, condensor and addition funnel were added 188.2g (2.0 mole) of phenol and 1.0g BF₃Et₂O catalyst. The reaction mixture was heated to 70 °C, and 17.4g (0.1 mole) of 5-(3-cyclohexen-1-yl)bicyclo[2.2.1]hept-2-ene was added over a 20-minute period. The temperature was raised to 150°C over a 1 1/2-hour period and held for 2 1/2 hours. Unreacted phenol was distilled. The recovered product had a melting range of 70-80 °C, a phenolic hydroxyl content of 0.495 eq/100g and a melt viscosity of 240 mPa.s (115 °C). The product polyphenol can be represented structurally as

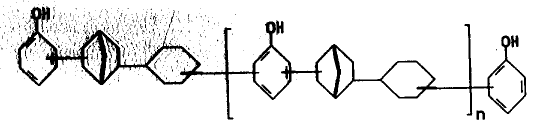

### Example 2

### Preparation of Polyphenol Based on Vinylcyclohexene/Cyclopentadiene Diadduct.

To a reactor equipped with a stirrer, condensor and addition funnel were added 376g (4.0 mole) of phenol and 2.0g of BF₃Et₂O catalyst. The reaction mixture was heated to 70 °C, and 48g (0.2 mole) of diadduct was added over a 20-minute period. The temperature was raised to 150 °C over a 1 1/2-hour period and held for about 2 1/2 hours. Unreacted phenol was distilled. The recovered product had a melting range of 85-95 °C. The product polyphenol can be represented structurally as

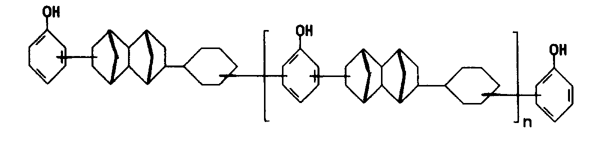

### Example 3

### Preparation of Polyphenol from Mixed Dienes.

To a reactor equipped with a stirrer, condensor and addition funnel were added 376g (4.0 mole) of phenol and 2.0g of BF₃Et₂O catalyst. The reaction mixture was heated to 70 °C, and 48g of a diene mixture obtained from the Diels-Alder reaction of cyclopentadiene and vinylcyclohexene were added over a 20-minute period. The temperature was raised to 150 °C over a 1 1/2-hour period and held for 2 1/2 hours. Unreacted phenol was distilled. The recovered product had a melting range of 87-100 °C. The product polyphenol includes repeating structural units

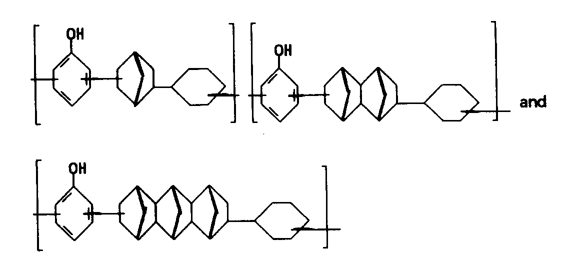

### Example 4

### Cure of Epoxy Resin.

27.5g of a 67/33 (wt) blend of the diglycidyl ether of bisphenol A and tetrabromo-BPA, 4.8g of the polyphenol prepared in Example 1 and 0.03g 2-imidazole were melt-blended at 150 °C. The mixture was then heated at 250 °C for 20 minutes. The resulting cured epoxy resin had a Tg of 91 °C.

### Example 5

### Cure of Epoxy Resin.

27.5g of a 67/33 (wt) blend of the diglycidyl ether of Example 2 and tetrabromo-BPA, 4.7g of the polyphenol prepared in Example 2 and 0.03g 2-imidazole were melt-blended at 150 °C. The mixture was then heated at 250 °C for 20 minutes. The resulting cured epoxy resin had a Tg of 91 °C.

### Example 6

### Cure of Epoxy Resin.

2g of the tetraglycidyl ether of the tetraphenol of ethane, 2g of the polyphenol prepared in Example 2 and 0.03g of 2-imidazole were melt-blended at 150 °C. The mixture was then heated at 250 °C for 20 minutes. The resulting cured epoxy resin had a Tg of 185 °C.

### Comparative Example 1

### Preparation of Polyphenol Based on Dicyclopentadiene (Comparison)

To a reactor equipped with a stirrer, condensor and addition funnel were added 188.2g (2.0 mole) of phenol and 1.0g of BF₃Et₂₀ catalyst. The reaction mixture was heated to 70 °C, and 13.2g (0.1 mole) of dicyclopentadiene was added over a 20-minute period and held for 2 1/2 hours. Unreacted phenol was distilled. The recovered product had a melting range of 115-120 °C, a phenolic hydroxyl content of 0.62 eq/100g, and a melt viscosity of 635 mPa.s (115 °C). The product can be represented structurally as

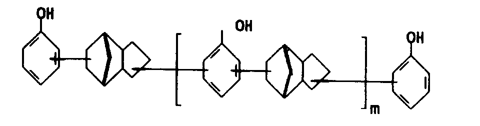

### Comparative Example 2

### Cure of Epoxy Resin (Comparison).

4.08g of a 67/33 (wt) blend of the diglycidyl ether of bisphenol A and tetrabromo-BPA, 1.61g of the polyphenol prepared in Comparative Example 1, and 0.03g of 2-imidazole were melt-blended at 150 °C. The mixture was then heated at 250 °C for 20 minutes. The resulting cured epoxy resin had a Tg of 118 °C.

### Example 7

### Preparation and cure of Epoxy resin B

A mixture of 220 of a polyphenol prepared by a process as described in Example 2 (hydroxyl content 0.44 eq/100g), 220g of epichlorohydrine and 4.4g of ethyltriphenylphosphonium bromide was placed in a 2L round-bottomed flask equipped with a mechanical stirrer and condensor.

The mixture was heated with stirring to 100 °C en was maintained at 100-110 °C for 4 hours.

The reaction mixture was then cooled to 80-90 °C. 150 Ml of toluene and 88g of 50% NaOH solution were added dropwise with distillation of H₂O. The toluene and excess epichlorohydrin were moved under reduced pressure to provide 248g of epoxy resin B isolated as a solid product, having a melting point of 65-70 °C and WPE of 342. Heating 59.26g of the product with 10.74 g of 4,4-diaminodiphenyl sulfone at 180 °C for 2 hours, 200 °C for 2 hours and 220 °C for 2 hours gave a cured material having the physical properties shown in Table 1. The product epoxy resin can be represented structurally as

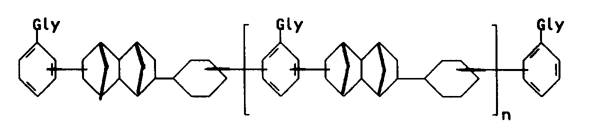

### Example 3

### Preparation and Cure of Epoxy Resin C (Comparison).

The procedure described in Example 7 was repeated starting with 25g of a polyphenol prepared by a process as described in Comparative Example 1 (0.62 eq. OH/100g), 41.3g ECH and 0.7g ETPPB. 25g of epoxy resin C (WPE 260) was isolated.

Curing 9.92g of the material with 2.37g of diaminodiphenyl sulfone according to the cure schedule described in Example 7 gave a cured resin having the physical properties shown in Table 1.

**Table 1**

| Comparison of Physical Properties of Resins A, B and C | | | | |
|---|---|---|---|---|
| | | A | B | C |
| Tg, °C | DSC | 180 | 198 | 196 |
| | DMA | 190 | 210 | 210 |

| Flex Properties, RT/Dry | | | | |
|---|---|---|---|---|
| Strength 10³.kPa (ksi) | | 126(18.3) | 124(18.0) | 141(20.5) |
| Modulus 10⁵.kPa (ksi) | | 32.9(478) | 30.9(442) | 30.5(442) |
| Elongation, % | | 4.3 | 4.5 | 5.7 |

| Flex Properties (Hot/Wet) | | | | |
|---|---|---|---|---|
| Strength 10³.kPa (ksi) | | 85.4(12.4) | 88.9(12.9) | 82.7(12.0) |
| Modulus 10⁵.kPa (ksi) | | 28.1(408) | 25.6(371) | 26.3(382) |
| Elongation, % | | 3.3 | 3.8 | 3.4 |
| Modulus Retention, % | | 85 | 84 | 86 |

| Fracture Toughness | | | | |
|---|---|---|---|---|
| 10⁶.kPa (cm)^{0.5}, (Kq) | | 11.7(463) | 12.3(486) | - |

| Moisture Gain, % | | | | |
|---|---|---|---|---|
| 200 h. | | 1.43 | 1.35 | 1.87 |
| 14 days | | 1.48 | 1.37 | 1.98 |
| Dielectric Constant | | 3.31 | 3.31 | - |
| Viscosity at 100 °C, mPa.s | | 170-180 | 1500 | 170-180 |

## Claims

1. A phenolic compound of the formula (I) 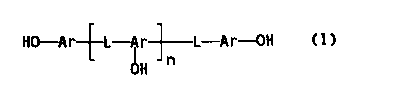 in which each Ar is a C₆₋₂₀ aromatic moiety, L is a divalent cyclohexane-norbornane moiety, and n is a number within the range of 0 to 10.

2. A phenolic compound as claimed in claim 1 in which Ar is phenyl and L is selected from at least one of the groups represented by the following structural formula:

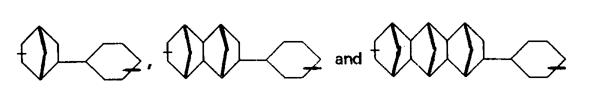

3. A composition comprising an epoxy resin and a phenolic compound as claimed in any one of the claims 1 and 2.

4. A process for preparing a phenolic compound as claimed in any one of the claims 1 to 2 by contacting in a reaction mixture at least one cyclohexenenorbornene compound with a molar excess, with respect to the cyclohexenenorbornene, of at least one phenolic compound in the presence of a Lewis acid addition catalyst at a temperature within the range of 70 to 200 °C.

5. A process as claimed in claim 4 wherein the cyclohexenenorbornene compound used comprises 5-(3-cyclohexen-1-yl)bicyclo[2.2.1]hept-2-ene.

6. An epoxy resin of the formula II 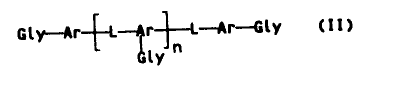 in which Gly is a glycidyl ether group, Ar is a C₆₋₂₀ aromatic moiety, L is a divalent cyclohexanenorbornane linking moiety, and n is a number within the range of 0 to 10.

7. An epoxy resin as claimed in claim 6 wherein Ar is phenyl and wherein L is selected from at least one of the groups represented by the following structural formula

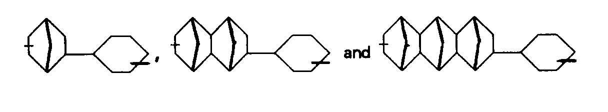

8. An epoxy resin composition comprising an epoxy resin as claimed in any one of claim 6 or 7 and an effective amount of a curing agent for the epoxy resin.

9. An epoxy resin composition as claimed in claim 8 wherein curing agent is diaminodiphenyl sulfone or methylenediamine.

10. A cured product obtainable by subjecting a composition as claimed in claims 3, 8 or 9 to a temperature of at least 150 °C for at least 0.25 hour.

11. An article comprising a cured product as claimed in claim 10.

## Patentansprüche

1. Phenolverbindung mit der Formel I 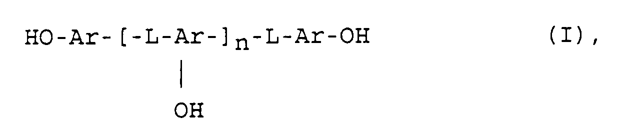 worin jedes Ar einen C₆₋₂₀ aromatischen Rest darstellt, L einen zweiwertigen Cyclohexannorbornanrest bedeutet und n eine Zahl im Bereich von 0 bis 10 ist.

2. Phenolverbindung nach Anspruch 1, worin Ar für Phenyl steht und L unter wenigstens einer der Gruppen ausgewählt ist, die durch die folgenden Strukturformeln dargestellt sind:

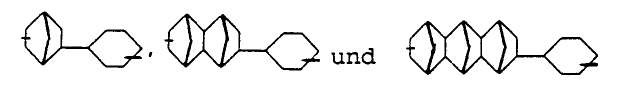

3. Zusammensetzung mit einem Gehalt an einem Epoxyharz und an einer Phenolverbindung nach einem der Ansprüche 1 und 2.

4. Verfahren zur Herstellung einer Phenolverbindung, wie in einem der Ansprüche 1 bis 2 beansprucht, durch Inkontaktbringen wenigstens einer Cyclohexennorbornenverbindung mit einem molaren Überschuß, bezogen auf das Cyclohexennorbornen, wenigstens einer Phenolverbindung in Anwesenheit eines Lewis-Säure-Additionskatalysators in einem Reaktionsgemisch bei einer Temperatur im Bereich von 70 bis 200°C.

5. Verfahren nach Anspruch 4, worin die verwendete Cyclohexennorbornenverbindung 5-(3-Cyclohexen-1-yl)bicyclo-[2.2.1.]hept-2-en umfaßt.

6. Epoxyharz mit der Formel II 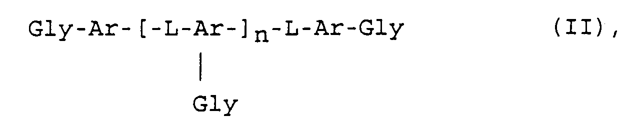 worin Gly eine Glycidylethergruppe bezeichnet, Ar für einen C₆₋₂₀ aromatischen Rest steht, L einen zweiwertigen Cyclohexannorbornanbrückenrest darstellt und n eine Zahl im Bereich von 0 bis 10 bedeutet.

7. Epoxyharz nach Anspruch 6, worin Ar Phenyl ist und worin L unter wenigstens einer der Gruppen ausgewählt ist, die durch die folgenden Strukturformeln dargestellt werden:

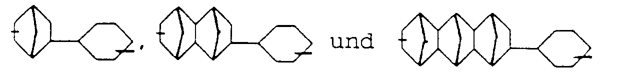

8. Epoxyharzzusammensetzung mit einem Gehalt an einem Epoxyharz, wie in einem der Ansprüche 6 oder 7 beansprucht, und an einer wirksamen Menge eines Härtungsmittels für das Epoxyharz.

9. Epoxyharzzusammensetzung nach Anspruch 8, worin das Härtungsmittel Diaminodiphenylsulfon oder Methylendiamin ist.

10. Gehärtetes Produkt, dadurch erhältlich, daß eine Zusammensetzung, wie in den Ansprüchen 3, 8 oder 9 beansprucht, einer Temperatur von wenigstens 150°C während wenigstens 0,25 Stunden ausgesetzt wird.

11. Gegenstand, umfassend ein gehärtetes Produkt nach Anspruch 10.

## Revendications

1. Composé phénolique de la formule I 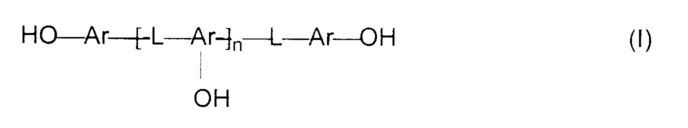 dans laquelle Ar représente un radical aromatique en C₆ à C₂₀, L représente un groupement cyclohexanenorbornane divalent et n est un nombre dont la valeur varie de 0 à 10.

2. Composé phénolique suivant la revendication 1, dans lequel Ar représente un radical phényle et L est choisi parmi au moins l'un des groupes représentés par les formules de structure suivantes :

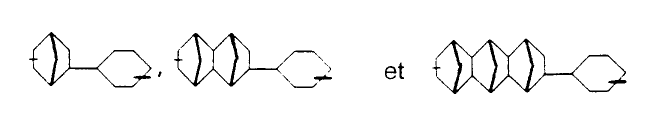

3. Composition comprenant une résine époxy et un composé phénolique suivant l'une quelconque des revendications 1 et 2.

4. Procédé de préparation d'un composé phénolique suivant l'une quelconque des revendications 1 et 2 par la mise en contact dans un mélange réactionnel d'au moins un composé de cyclohexènenorbornène avec un excès molaire, par rapport au cyclohexènenorbornène, d'au moins un composé phénolique, en présence d'un catalyseur d'addition du type acide de Lewis, à une température qui varie de 70 à 200°C.

5. Procédé suivant la revendication 4, caractérisé en ce que le composé de cyclohexènenorbornène utilisé est constitué de 5-(3-cyclohexène-1-yl)bicyclo[2.2.1]hept-2-ène.

6. Résine époxy de la formule II 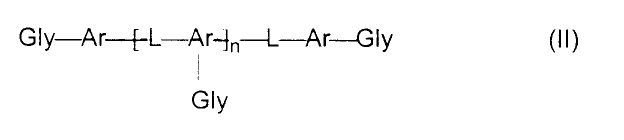 dans laquelle Gly représente un radical éther glycidylique, Ar représente un groupement aromatique en C₆ à C₂₀, L représente un groupement de liaison du type cyclohexanenorbornane divalent et n est un nombre dont la valeur varie de 0 à 10.

7. Résine époxy suivant la revendication 6, caractérisée en ce que Ar représente un radical phényle et L est choisi parmi au moins l'un des radicaux représentés par les formules de structure suivantes

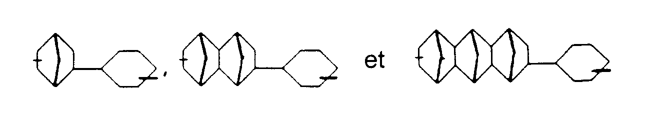

8. Composition de résine époxy comprenant une résine époxy, telle que revendiquée dans l'une quelconque des revendications 6 et 7 et une quantité efficace d'un agent de durcissement pour la résine époxy.

9. Composition de résine époxy suivant la revendication 8, caractérisée en ce que l'agent de durcissement est la diaminodiphénylsulfone ou la méthylènediamine.

10. Produit durci que l'on peut obtenir en soumettant une composition telle que revendiquée dans les revendications 3, 8 ou 9, à une température d'au moins 150°C, pendant au moins 0,25 heure.

11. Article comprenant un produit durci suivant la revendication 10.
